# EUROPÄISCHE PATENTSCHRIFT

(11) **EP 1 374 823 B1**
(45) Veröffentlichungstag und Bekanntmachung des Hinweises auf die Patenterteilung: **07.06.2006**
(21) Anmeldenummer: 03008286.1
(22) Anmeldetag: 09.04.2003
(51) Int. Cl.: A61G 13/10, F16M 13/02

(54) **Befestigungsstück zur Halterung von Zubehörteilen an der Lagerungsfläche eines Operationstisches**
Fastener for attaching accessories to the lying surface of a surgical table
Elément de fixation pour attacher des accessoires au surface de couchage d'une table d'opération

(30) Priorität: 17.06.2002 DE 20209387 U
(43) Veröffentlichungstag der Anmeldung: 02.01.2004
(73) Patentinhaber: MAQUET GmbH & Co. KG, 76437 Rastatt (DE)
(72) Erfinder: Pfeuffer, Reinhard, 76477 Elchesheim-Illingen (DE); Ropertz, Frank, 76530 Baden-Baden (DE)
(74) Vertreter: Schaumburg, Thoenes, Thurn, Landskron

(56) Entgegenhaltungen:
- WO-A-98/49414
- US-A- 1 266 367
- US-A- 4 592 526

## Beschreibung

Die Erfindung betrifft ein Befestigungsstück zur Halterung von Zubehörteilen an der Lagerfläche eines Operationstisches oder dergleichen gemäß dem Oberbegriff des Anspruchs 1.

Ein Befestigungsstück dieser Art ist aus der US-A-45 92 526 bekannt. Bei dieser Lösung werden die Zahnscheiben jeweils durch einen beide Zahnscheiben durchsetzenden Gewindebolzen mit Mutter zusammengespannt. Für das Lockern und Festziehen der Mutter ist Werkzeug erforderlich.

Der Erfindung liegt die Aufgabe zugrunde, ein Befestigungsstück der eingangs genannten Art anzugeben, das einen großen Verstellbereich gewährleistet, einfach zu verstellen ist und zuverlässig in der einmal gewählten Stellung arretiert werden kann, wobei ein unbeabsichtigtes Lösen der Arretierung weitgehend ausgeschlossen sein soll.

Diese Aufgabe wird erfindungsgemäß durch die im kennzeichnenden Teil des Anspruchs 1 angegebenen Merkmale gelöst.

Die miteinander in Eingriff tretenden Zahnringe der Zahnscheiben gewährleisten eine absolut zuverlässige Arretierung des Befestigungsstückes in der einmal gewählten Stellung. Jedes Gelenk kann separat für sich verstellt werden. Die Feinfühligkeit der Verstellung hängt von der Zahnteilung ab. Der Schwenkbereich der in einem Gelenk miteinander verbundenen Teile relativ zueinander ist sehr groß und kann besonders groß gemacht werden, wenn das mit der jeweiligen Zahnscheibe verbundene Teil an der dem Zahnring abgewandten Seite der Zahnscheibe befestigt ist. Die Spannschraube ist in der Weise ausgebildet, daß eine der Zahnscheiben jedes Gelenks starr mit einem Schraubbolzen und die andere Zahnscheibe mit einer eine Durchtrittsbohrung für den Schraubbolzen aufweisenden Buchse verbunden ist, an deren axialer Endfläche sich eine auf den Schraubbolzen aufschraubbare Mutter abstützt, die zur leichteren Betätigung mit einem Drehgriff verbunden ist.

Da die Zahnscheiben durch zwischen ihnen angeordnete Federmittel axial voneinander weg vorgespannt sind, beispielsweise durch eine Schraubendruckfeder oder eine Tellerfeder, wird gewährleistet, daß die Zahnscheiben beim Lockern der Spannschraube automatisch axial auseinandertreten, so daß die durch das Gelenk miteinander verbunden Teile gegeneinander verschwenkt werden können.

Um auch hohe Spannkräfte mit geringer Reibung aufbringen zu können, ist es zweckmäßig, wenn zwischen der Mutter und der axialen Endfläche der Buchse ein Axiallager angeordnet ist.

Zweckmäßigerweise trägt ein erster Gelenkhebel an jedem seiner Enden eine Zahnscheibe mit einem Schraubbolzen, während der andere Gelenkhebel an jedem seiner Enden eine Zahnscheibe mit einer Buchse trägt. Diese Anordnung sorgt dafür, daß sämtliche Muttern bzw. mit ihnen verbundene Drehgriffe auf einer Seite des Befestigungsstückes liegen, wodurch die Einstellung der Position des Befestigungsstückes erleichtert wird.

Das Anschlußteil kann in an sich bekannter Weise als Steckschiene ausgebildet sein, die in eine Führung an der Lagerfläche des Op-Tisches einsteckbar und in der Führung arretierbar ist. Die Profilschiene ist zweckmäßigerweise mit ihrer Längsrichtung parallel zu den Gelenkachsen gerichtet.

Das vorstehend beschriebene Befestigungsstück ermöglicht eine feinfühlige Verstellung der Profilschiene relativ zu der Lagerfläche eines Operationstisches über einen großen Verstelllbereich und eine absolut sichere Arretierung des Befestigungsstückes in der einmal eingestellten Position.

Die folgende Beschreibung erläutert in Verbindung mit den beigefügten Zeichnungen die Erfindung anhand eines Ausführungsbeispieles. Es zeigen:
- Figur 1: eine perspektivische Gesamtansicht des erfindungsgemäßen Befestigungsstückes,
- Figur 2: eine Draufsicht auf das in Figur 1 dargestellte Befestigungsstück und
- Figur 3: einen Schnitt durch das profilschienenseitige Gelenk entlang der Linie lll-lll von Figur 1.

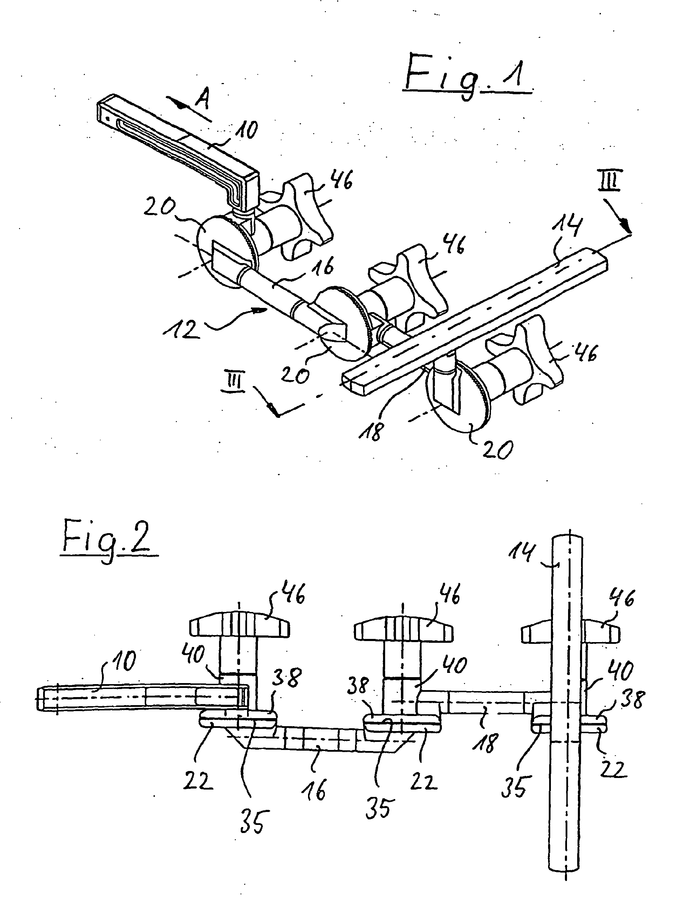

Das in der Figur 1 dargestellte Befestigungsstück umfaßt ein als Steckschiene ausgebildetes Anschlußteil 10, das in Richtung des Pfeiles A in eine geeignete Führung oder Steckhülse an der Lagerfläche eines Operationstisches oder dergleichen einsteckbar ist, einen allgemein mit 12 bezeichneten Gelenkarm und eine Profilschiene 14, die durch den Gelenkarm 12 mit dem Anschlußstück 10 gelenkig verbunden ist und die zum Anklemmen eines Zubehörteiles, beispielsweise einer Kopfplatte für den Kopf eines Patienten bestimmt ist.

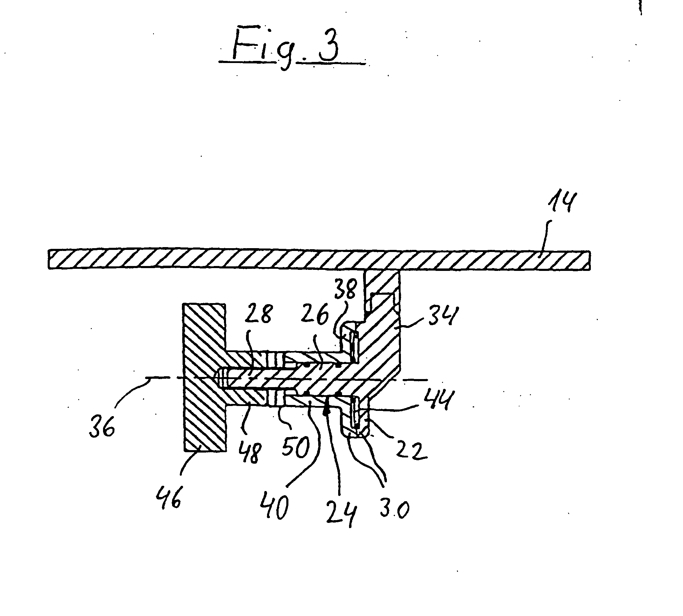

Der Gelenkarm 12 hat zwei stangenförmige Gelenkhebel 16, 18, die über ein erstes Gelenk 20 miteinander verbunden sind. Der erste Gelenkhebel 16 ist über ein weiteres Gelenk 20 mit dem Anschlußteil 10 verbunden, während das andere Ende des zweiten Gelenkhebels 18 über ein weiteres Gelenk 20 mit der Profilschiene 14 verbunden ist. Alle drei Gelenke 20 haben einen gleichen Aufbau, der anhand der Figur 3 näher erläutert werden soll. Jedes Gelenk umfaßt eine erste Zahnscheibe 22, die mit einem co-axial zur Gelenkachse gerichteten, als Spannschraube ausgebildete Gelenkbolzen 24 einstückig ausgebildet ist. Der Gelenkbolzen hat ein glattes zylindrisches Schaftteil 26 und einen durchmesserkleineren Gewindeabschnitt 28. Die Zahnscheibe 22 hat einen an ihrem radial äußeren Rand umlaufenden Zahnring oder Zahnkranz 30 mit einer axial gerichteten Verzahnung 32 (Figur 2). Auf ihrer dem Zahnkranz abgewandten Seite ist die Zahnscheibe 22 mit einem Ansatz 34 verbunden, der die Profilschiene 40 trägt, die mit ihrer Längsrichtung parallel zur Gelenkachse 36 gerichtet ist.

Der Zahnscheibe 22 ist eine zweite Zahnscheibe 38 zugeordnet, die auf ihrer der Zahnscheibe 22 zugewandten achsnormalen Fläche ebenfalls einen Zahnring oder Zahnkranz 30 mit axial gerichteter Verzahnung hat. Auf ihrer dem Zahnkranz 30 abgewandten Seite ist die Zahnscheibe 38 mit einer Buchse 40 verbunden, welche den Schaftteil 26 des Gelenkbolzens 24 in einer Bohrung 42 aufnimmt. Zwischen den beiden Zahnscheiben 22 und 38 ist durch die axial vorstehenden Zahnkränze 30 ein Hohlraum gebildet, in dem sich eine Tellerfeder 44 befindet, welche die Zahnscheiben 22 und 38 axial auseinanderdrückt. Auf den Gewindeabschnitt 28 des Gelenkbolzens 24 ist eine in Form eines Drehgriffes 46 verbundene Mutter 48 aufgeschraubt, mit der die beiden Zahnscheiben 22 und 38 gegeneinander gespannt werden können. Um die Reibung zwischen der Mutter 48 und der axialen Endfläche der Buchse 40 zu verringern, ist zwischen der Buchse 40 und der Mutter 48 ein Axiallager 50 angeordnet.

Wie man in den Figuren 1 und 2 erkennt, ist die Ausbildung so getroffen, daß der erste Gelenkhebel 16 an jedem seiner Enden mit einer einen Gelenkbolzen 24 tragenden Zahnscheibe 22 verbunden. Der zweite Gelenkhebel 18 dagegen ist jeweils mit einer eine Buchse 40 tragenden Zahnscheibe 38 verbunden. Das Anschlußstück 10 ist mit einer Buchse 40 tragenden Zahnscheibe verbunden, während die Profilschiene 14 wiederum mit einer einen Gelenkbolzen 24 tragenden Zahnscheibe 22 verbunden ist, wie dies bereits anhand der Figur 3 erläutert wurde. Diese Anordnung führt dazu, daß alle drei Drehgriffe 46 auf derselben Seite des Befestigungsstückes liegen, wie dies die Figuren 1 und 2 zeigen, so daß die Verstellung der Abschnitte des Betätigungsstückes in den drei Gelenken bequem aus derselben Position der Bedienungsperson heraus erfolgen kann, ohne daß diese umgreifen muß.

Die vorstehende Beschreibung zeigt, daß das erfindungsgemäße Befestigungsstück bei einem großen Verstellbereich feinfühlig verstellt und absolut zuverlässig in der gewünschten Position arretiert werden kann, wobei die unabsichtliche Lockerung der Arretierung praktisch ausgeschlossen ist.

## Patentansprüche

1. Befestigungsstück zur Halterung von Zubehörteilen an der Lagerfläche eines Operationstisches oder dergleichen mit einem Anschlußteil (10) zur Verbindung des Befestigungsstückes mit der Lagerfläche einer Profilschiene (14) zum Befestigen eines Zubehörteiles und einem die Profilschiene (14) mit dem Anschlußteil (10) verbindenden Gelenkarm (12) mit mindestens zwei Gelenkhebeln (16, 18), die untereinander sowie mit dem Anschlußteil (10) einerseits und der Profilschiene (14) andererseits über jeweils ein feststellbares Gelenk (20) verbunden sind, wobei die Gelenkachsen parallel zueinander sind, jedes Gelenk zwei zueinander co-axiale Zahnscheiben (22, 38) hat, die an ihren einander zugekehrten Flächen jeweils einen zur Gelenkachse co-axialen Zahnring (30) haben, jede Zahnscheibe (22, 38) starr mit einem der durch ein Gelenk (20) miteinander zu verbindenden Teile (10, 16, 18, 14) verbunden ist und wobei die Zahnscheiben (22, 38) jedes Gelenks durch eine zur jeweiligen Gelenkachse co-axiale Spannschraube (24) mit Mutter (48) gegeneinander spannbar sind, um die Zahnringe (30) der gegeneinander gespannten Zahnscheiben (22, 38) in Eingriff miteinander zu bringen, **dadurch gekennzeichnet, daß** eine der Zahnscheiben (22) jedes Gelenkes starr mit der Spannschraube (24) und die andere Zahnscheibe (38) mit einer eine Durchtrittsbohrung (42) für den Schraubbolzen (24) aufweisenden Buchse (40) verbunden ist, an deren axialer Endfläche sich die auf den Schraubbolzen (24) aufschraubbare Mutter (48) abstützt, die mit einem Drehgriff (46) verbunden ist, und dass die Zahnscheiben (22, 38) durch zwischen ihnen angeordnete Federmittel (44) axial voneinander weg vorgespannt sind.

2. Befestigungsstück nach Anspruch 1, **dadurch gekennzeichnet, daß** das mit der jeweiligen Zahnscheibe (22, 38) verbundene Teil (10, 16, 18, 14) an der dem Zahnring (30) abgewandten Seite der Zahnscheibe (22, 38) befestigt ist.

3. Befestigungsstück nach Anspruch 1 oder 2, **dadurch gekennzeichnet, daß** zwischen der Mutter (48) und der axialen Endfläche der Buchse (40) ein Axiallager (50) angeordnet ist.

4. Befestigungsstück nach einem der Ansprüche 1 bis 3, **dadurch gekennzeichnet, daß** ein erster Gelenkhebel an jedem seiner Enden eine Zahnscheibe (22) mit einem Schraubbolzen (24) trägt und daß der zweite Gelenkhebel (18) an jedem seiner Enden eine Zahnscheibe (38) mit einer Buchse (40) trägt.

5. Befestigungsstück nach einem der Ansprüche 1 bis 4, **dadurch gekennzeichnet, daß** das Anschlußteil (10) als Steckschiene ausgebildet ist, die in eine Führung an der Lagerfläche des Operationstisches einsteckbar und in der Führung arretierbar ist.

6. Befestigungsstück nach einem der Ansprüche 1 bis 5, **dadurch gekennzeichnet, daß** die Profilschiene (14) mit ihrer Längsrichtung parallel zu den Gelenkachsen (36) gerichtet ist.

## Claims

1. Fastener for attaching accessories to the lying surface of a surgical table or similar with a connecting part (10) for connecting the fastener to the lying surface, a profiled track (14) for fastening an accessory and a hinged arm (12) connecting the profiled track (14) to the connecting part (10) having at least two articulated levers (16, 18) which are connected together and to the connecting part (10) on the one hand and the profiled track (14) on the other hand by means of a fixable hinge (20) in each case, wherein the hinge axes are parallel to each other, each hinge has two tooth washers (22, 38) which are coaxial with each other and have on their respective surfaces facing each other an annular gear (30) which is coaxial with the hinge axis, each tooth washer (22, 38) is rigidly connected to one of the parts (10, 16, 18, 14) to be connected to each other by a hinge (20) and wherein the tooth washers (22, 38) of each hinge are adapted to be clamped against each other by a clamping screw (24) with a nut (48) coaxial with the respective hinge axis in order to bring the annular gears (30) of the tooth washers (22, 38) clamped against each other into engagement with each other, **characterised in that** one of the tooth washers (22) of each hinge is rigidly connected to the clamping screw (24) and the other tooth washer (38) is connected to a socket (40) having a passage bore (42) for the screw bolt (24), on the axial end surface of which socket (40) the nut (48) which is adapted to be screwed onto the screw bolt (24) rests, which nut (48) is connected to a rotary grip (46) and **in that** the tooth washers (22, 38) are pre-tensioned axially away from each other by spring means (44) arranged between them.

2. Fastener according to claim 1, **characterised in that** the part (10, 16, 18, 14) connected to the respective tooth washer (22, 28) is secured to the side of the tooth washer (22, 38) facing away from the annular gear (30).

3. Fastener according to claim 1 or 2, **characterised in that** an axial bearing (50) is disposed between the nut (48) and the axial end surface of the socket (40).

4. Fastener according to one of the claims 1 to 3, **characterised in that** a first articulated lever carries on each of its ends a tooth washer (22) with a screw bolt (24) and **in that** the second articulated lever (18) carries a tooth washer (38) with a socket (40) on each of its ends.

5. Fastener according to one of the claims 1 to 4, **characterised in that** the connecting part (10) is formed as an insert track which is adapted to be inserted into a guide on the lying surface of the surgical table and locked in the guide.

6. Fastener according to one of the claims 1 to 5, **characterised in that** the profiled track (14) is arranged with its longitudinal direction parallel to the hinge axes (36).

## Revendications

1. Pièce de fixation destinée à tenir des accessoires au niveau de la surface porteuse d'une table d'opération ou analogue, ladite pièce de fixation comportant une pièce de raccordement (10) destinée à relier la pièce de fixation à la surface porteuse, un rail profilé (14) destiné à fixer un accessoire, et un bras articulé (12) qui relie le rail profilé (14) à la pièce de raccordement (10) et qui comporte au moins deux leviers articulés (16, 18) qui sont reliés entre eux ainsi qu'à la pièce de raccordement (10) d'une part et au rail profilé (14) à chaque fois par une articulation blocable (20), les axes d'articulation étant parallèles entre eux, et chaque articulation comportant deux disques dentés coaxiaux (22, 38) qui comportent chacun sur leurs surfaces dirigées l'une vers l'autre une couronne dentée (30) coaxiale à l'axe d'articulation, chaque disque denté (22, 38) étant relié rigidement à l'une des pièces (10, 16, 18, 14) qui doivent être reliées entre elles par une articulation (20) et les disques dentés (22, 38) de chaque articulation pouvant être serrés l'un contre l'autre par une vis de serrage (24) coaxiale à l'axe d'articulation respectif et dotée d'un écrou (48) afin d'amener en prise l'une avec l'autre les couronnes dentées (30) des disques dentés (22, 38) serrés l'un contre l'autre, **caractérisée en ce que** l'un des disques dentés (22) de chaque articulation est relié rigidement à la vis de serrage (24) tandis que l'autre disque denté (38) est relié rigidement à une douille (40) qui comporte un perçage traversant (42) destiné au boulon fileté (24) et sur la surface d'extrémité de laquelle vient s'appuyer l'écrou (48) qui peut se visser sur le boulon fileté (24) et qui est relié à une poignée tournante (46), et **en ce que** les disques dentés (22, 38) sont précontraints axialement en éloignement l'un de l'autre par un moyen élastique (44) disposé entre eux.

2. Pièce de fixation selon la revendication 1, **caractérisée en ce que** la pièce (10, 16, 18, 14), reliée au disque denté respectif (22, 38), est fixée au côté, opposé à la couronne dentée (30), du disque denté (22, 38).

3. Pièce de fixation selon la revendication 1 ou 2, **caractérisée en ce qu'**un palier axial (50) est disposé entre l'écrou (48) et la surface d'extrémité axiale de la douille (40).

4. Pièce de fixation selon l'une des revendications 1 à 3, **caractérisée en ce qu'**un premier levier articulé porte à chacune de ses extrémités un disque denté (22) doté d'un boulon fileté (24), et **en ce que** le deuxième levier articulé (18) porte à chacune de ses extrémités un disque denté (38) doté d'une douille (40).

5. Pièce de fixation selon l'une des revendications 1 à 4, **caractérisée en ce que** la pièce de raccordement (10) est conformée en rail à enficher qui est enfichable dans un guide ménagé au niveau de la surface porteuse de la table d'opération, et qui est arrêtable dans le guide.

6. Pièce de fixation selon l'une des revendications 1 à 5, **caractérisée en ce que** la direction longitudinale du rail profilé (14) est parallèle aux axes d'articulation (36).
